# FASCICULE DE BREVET EUROPEEN

(11) **EP 0 848 946 B1**
(45) Date de publication et mention de la délivrance du brevet: **30.10.2002**
(21) Numéro de dépôt: 97402715.3
(22) Date de dépôt: 13.11.1997
(51) Int. Cl.: A61K 7/42

(54) **Compositions comprenant un dérivé de dibenzoylméthane, un dérivé de 1,3,5-triazine et un composé amide et utilisations**
Zusammensetzungen enthaltend ein Dibenzoylmethanderivat, ein 1,3,5-Triazinderivat und eine Amid-Verbindung sowie ihre Verwendung
Compositions comprising a dibenzoylmethane derivative, a 1,3,5 -triazine derivative and an amide derivative and their use

(30) Priorité: 17.12.1996 FR 9615513
(43) Date de publication de la demande: 24.06.1998
(73) Titulaire: L'OREAL, 75008 Paris (FR)
(72) Inventeur: Pisson, Anne-Marie, 91800 Brunoy (FR); Allard, Delphine, 92700 Colombes (FR); Candau, Didier, 91570 Bievres (FR)
(74) Mandataire: Miszputen, Laurent

(56) Documents cités:
- EP-A- 0 457 687
- EP-A- 0 517 104
- EP-A- 0 689 828
- EP-A- 0 717 982
- EP-A- 0 748 623

## Description

La présente invention concerne de nouvelles compositions cosmétiques et/ou dermatologiques (ci-après appelées compositions antisolaires) destinées à la protection de la peau et/ou des cheveux contre les rayonnements UV, en particulier le rayonnement solaire. Plus précisément, elle concerne de nouvelles compositions cosmétiques et/ou dermatologiques présentant une photostabilité améliorée et comprenant, dans un support cosmétiquement et/ou dermatologiquement acceptable, l'association de trois composés particuliers.

L'invention concerne également l'utilisation de ces compositions dans les domaines cosmétique et/ou dermatologique.

On sait que les radiations lumineuses de longueurs d'onde comprises entre 280 nm et 400 nm permettent le brunissement de l'épiderme humain, et que les rayons de longueurs d'onde plus particulièrement comprises entre 280 et 320 nm, connus sous la dénomination d'UV-B, provoquent des érythèmes et des brûlures cutanées qui peuvent nuire au développement du bronzage naturel. Pour ces raisons ainsi que pour des raisons esthétiques, il existe une demande constante de moyens de contrôle de ce bronzage naturel en vue de contrôler ainsi la couleur de la peau ; il convient donc de filtrer ce rayonnement UV-B.

On sait également que les rayons UV-A, de longueurs d'onde comprises entre 320 et 400 nm, qui provoquent le brunissement de la peau, sont susceptibles d'induire une altération de celle-ci, notamment dans le cas d'une peau sensible ou d'une peau continuellement exposée au rayonnement solaire. Les rayons UV-A provoquent en particulier une perte d'élasticité de la peau et l'apparition de rides conduisant à un vieillissement cutané prématuré. Ils favorisent le déclenchement de la réaction érythémateuse ou amplifient cette réaction chez certains sujets et peuvent même être à l'origine de réactions phototoxiques ou photo-allergiques. Ainsi, pour des raisons esthétiques et cosmétiques telles que la conservation de l'élasticité naturelle de la peau par exemple, de plus en plus de gens désirent contrôler l'effet des rayons UV-A sur leur peau. Il est donc souhaitable de filtrer aussi le rayonnement UV-A.

Ainsi, dans le but d'assurer une protection de la peau et des cheveux contre l'ensemble du rayonnement UV qui soit la plus complète et la plus efficace possible, on utilise généralement dans la fabrication des compositions antisolaires des associations de filtres actifs dans l'UVA et de filtres actifs dans l'UVB.

A cet égard, une famille de filtres UV-A particulièrement intéressante est actuellement constituée par les dérivés du dibenzoylméthane, et notamment le 4-(ter.-butyl) 4'-méthoxy dibenzoylméthane, qui présentent en effet un fort pouvoir d'absorption intrinsèque. Ces dérivés du dibenzoylméthane, qui sont maintenant des produits bien connus en soi à titre de filtres actifs dans l'UV-A, sont notamment décrits dans les demandes de brevets français FR-A-2326405 et FR-A-2440933, ainsi que dans la demande de brevet européen EP-A-0114607 ; le 4-(ter.-butyl) 4'-méthoxy dibenzoylméthane est par ailleurs actuellement proposé à la vente sous la dénomination commerciale de "PARSOL 1789" par la Société GIVAUDAN.

De même, les dérivés de 1,3,5-triazine, et en particulier la 2,4,6-tris[p-(2'-éthylhexyl-1'-oxycarbonyl)anilino]-1,3,5-triazine, vendue sous la dénomination commerciale « UVINUL T150 » par la société BASF, possèdent un fort pouvoir absorbant des UVB et il serait donc très intéressant de pouvoir les utiliser en association avec le 4-tert-butyl-4'-méthoxydibenzoylméthane cité ci-dessus dans le but d'obtenir des produits offrant une protection large et efficace dans l'ensemble du rayonnement UV.

Toutefois, la Demanderesse a constaté que ces dérivés de 1,3,5-triazine, lorsqu'ils sont en présence de dérivés du dibenzoylméthane, en particulier du 4-tert-butyl-4'-méthoxydibenzoylméthane, et sous irradiation UV, présentent l'inconvénient de se dégrader chimiquement de façon importante. Dans ces conditions, l'association des deux filtres ne permet plus une protection solaire large prolongée de la peau et des cheveux.

Or, à la suite d'importantes recherches menées dans le domaine de la photoprotection évoqué ci-dessus, la Demanderesse a maintenant découvert que l'introduction d'un composé amidé dans une composition contenant un dérivé de dibenzoylméthane, en particulier du 4-tert-butyl-4'-méthoxydibenzoylméthane, en association avec au moins un dérivé de 1,3,5-triazine, et en particulier avec la 2,4,6-tris[p-(2'-éthylhexyl-1'-oxycarbonyl)anilino]-1,3,5-triazine, permettait d'améliorer de façon tout à fait remarquable la photostabilité de ce dérivé de 1,3,5-triazine au sein de telles compositions, et donc l'efficacité globale de ces compositions.

La présente invention a donc pour objet de nouvelles compositions cosmétiques et/ou dermatologiques comprenant, dans un support cosmétiquement et/ou dermatologiquement acceptable :
- i) au moins un dérivé de 1,3,5-triazine répondant à la formule (I) suivante : 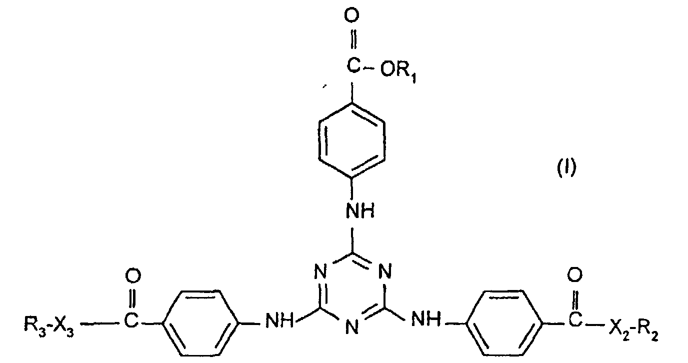 dans laquelle :
   - X₂ et X₃, identiques ou différents, représentent l'oxygène ou le radical -NH- ;
   - R₁, R₂ et R₃, identiques ou différents, sont choisis parmi : l'hydrogène; un métal alcalin ; un radical ammonium éventuellement substitué par un ou plusieurs radicaux alkyles ou hydroxyalkyles; un radical alkyle linéaire ou ramifié en C₁-C₁₈ ; un radical cycloalkyle en C₅-C₁₂ éventuellement substitué par un ou plusieurs radicaux alkyles en C₁-C₄ ; un radical polyoxyéthyléné comprenant de 1 à 6 unités d'oxyde d'éthylène et dont le groupe OH terminal est méthylé; un radical de formule (II), (III) ou (IV) suivantes : 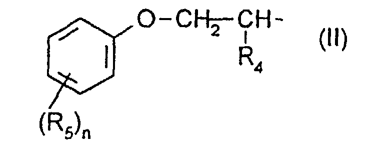 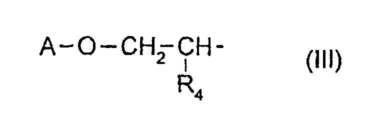 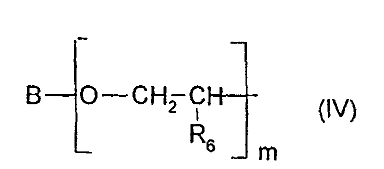
   dans lesquelles :
   - R₄ est l'hydrogène ou un radical méthyle;
   - R₅ est un radical alkyle en C₁-C₉;
   - n est un nombre entier allant de 0 à 3;
   - m est un nombre entier allant de 1 à 10;
   - A est un radical alkyle en C₄-C₈ ou un radical cycloalkyle en C₅-C₈;
   - B est choisi parmi : un radical alkyle linéaire ou ramifié en C₁-C₈; un radical cycloalkyle en C₅-C₈; un radical aryle éventuellement substitué par un ou plusieurs radicaux alkyles en C₁-C₄;
   - R₆ est l'hydrogène ou un radical méthyle,
- ii) au moins un dérivé de dibenzoylméthane répondant à la formule (V) suivante : 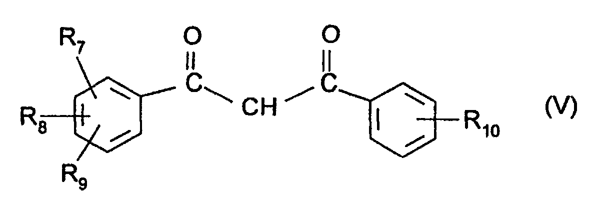 dans laquelle R₇, R₈, R₉ et R₁₀, identiques ou différents, représentent indépendamment l'hydrogène ou un radical hydroxyle ou un radical alkyle linéaire ou ramifié en C₁-C₈ ou un radical alcoxy linéaire ou ramifié en C₁-C₈,
- et iii) au moins un composé amidé.

Ainsi, selon la présente invention, on peut réaliser des compositions cosmétiques et/ou dermatologiques contenant un dérivé de dibenzoylméthane, en particulier du 4-tert-butyl-4'-méthoxydibenzoylméthane, en association avec au moins un dérivé de 1,3,5-triazine, compositions dans lesquelles la concentration en dérivé de 1,3,5-triazine reste relativement constante même si ces compositions sont soumises à l'action de la lumière.

La présente invention a encore pour objet l'utilisation d'un composé amidé dans des compositions cosmétiques et/ou dermatologiques contenant un dérivé de dibenzoylméthane tel que défini ci-dessus, en particulier du 4-tert-butyl-4'-méthoxydibenzoylméthane, en association avec au moins un dérivé de 1,3,5-triazine tel que défini ci-dessus en vue d'améliorer dans lesdites compositions la stabilité au rayonnement UV (photostabilité) dudit dérivé de 1,3,5-triazine.

La présente invention a également pour objet un procédé pour améliorer la stabilité au rayonnement UV (photostabilité), et donc l'efficacité, d'une composition cosmétique et/ou dermatologique comprenant un dérivé de dibenzoylméthane tel que défini ci-dessus, en particulier du 4-tert-butyl-4'-méthoxydibenzoylméthane, et un dérivé de 1,3,5-triazine tel que défini ci-dessus, en particulier la 2,4,6-tris[p-(2'-éthylhexyl-1'-oxycarbonyl)anilino]-1,3,5-triazine, ledit procédé consistant à introduire dans ladite composition une quantité efficace d'un composé amidé.

Par quantité efficace de composé amidé, on entend une quantité suffisante pour obtenir une amélioration notable et significative de la photostabilité du ou des dérivés du dibenzoylméthane contenus dans la composition. Cette quantité minimale en agent stabilisant à mettre en oeuvre, qui peut varier selon la nature du support cosmétiquement acceptable retenu pour la composition, peut être déterminée sans aucune difficulté au moyen d'un test classique de mesure de photostabilité, tel que celui donné dans les exemples ci-après.

D'autres caractéristiques, aspects et avantages de la présente invention apparaîtront à la lecture de la description détaillée qui va suivre.

Un premier composé des compositions visées par la présente invention est un dérivé particulier de 1,3,5-triazine. Ainsi, les dérivés de 1,3,5-triazine utilisables dans le cadre de la présente invention sont choisis parmi ceux répondant à la formule (I) suivante : 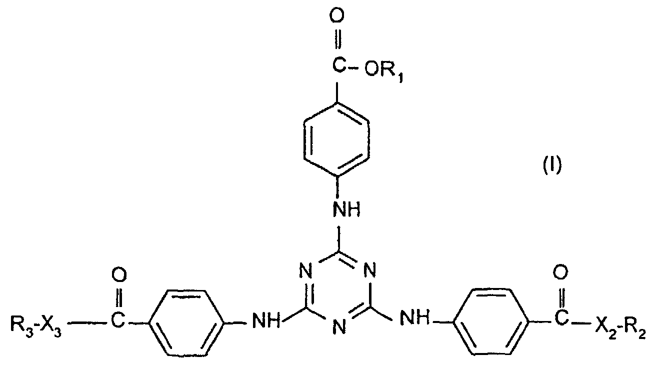 dans laquelle :
- X₂ et X₃, identiques ou différents, représentent l'oxygène ou le radical -NH- ;
- R₁, R₂ et R₃, identiques ou différents, sont choisis parmi : l'hydrogène; un métal alcalin ; un radical ammonium éventuellement substitué par un ou plusieurs radicaux alkyles ou hydroxyalkyles ; un radical alkyle linéaire ou ramifié en C₁-C₁₈ ; un radical cycloalkyle en C₅-C₁₂ éventuellement substitué par un ou plusieurs radicaux alkyles en C₁-C₄ ; un radical polyoxyéthyléné comprenant de 1 à 6 unités d'oxyde d'éthylène et dont le groupe OH terminal est méthylé; un radical de formule (II), (III) ou (IV) suivantes : 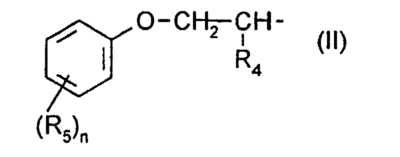 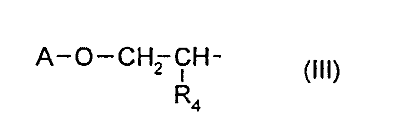 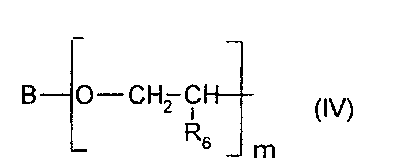 dans lesquelles :
   - R₄ est l'hydrogène ou un radical méthyle ;
   - R₅ est un radical alkyle en C₁-C₉ ;
   - n est un nombre entier allant de 0 à 3 ;
   - m est un nombre entier allant de 1 à 10 ;
   - A est un radical alkyle en C₄-C₈ ou un radical cycloalkyle en C₅-C₈ ;
   - B est choisi parmi : un radical alkyle linéaire ou ramifié en C₁-C₈ ; un radical cycloalkyle en C₅-C₈ ; un radical aryle éventuellement substitué par un ou plusieurs radicaux alkyles en C₁-C₄ ;
   - R₆ est l'hydrogène ou un radical méthyle.

Bien entendu, dans la définition ci-dessus, lorsque X₂ et/ou X₃ représentent un radical -NH-, alors le ou les radicaux R₂ et/ou R₃ correspondants sont différents d'un métal alcalin ou d'un radical ammonium.

Une première famille plus particulièrement préférée de dérivés de 1,3,5-triazine est celle, notamment décrite dans le document EP-A-0517104, des 1,3,5-triazines répondant à la formule (I) ci-dessus et présentant l'ensemble des caractéristiques suivantes :
- X₂ et X₃ sont identiques et représentent l'oxygène ;
- R₁ est choisi parmi : un radical cycloalkyle en C₅-C₁₂ éventuellement substitué par un ou plusieurs radicaux alkyles en C₁-C₄ ; un radical de formule (II), (III) ou (IV) ci-dessus dans lesquelles :
   - B est un radical alkyle en C₁-C₄ ;
   - R₆ est le radical méthyle ;
- R₂ et R₃, identiques ou différents, sont choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈ ; un radical cycloalkyle en C₅-C₁₂ éventuellement substitué par un ou plusieurs radicaux alkyles en C₁-C₄ ; un radical de formule (II), (III) ou (IV) ci-dessus dans lesquelles :
   - B est un radical alkyle en C₁-C₄ ;
   - R₆ est le radical méthyle.

Une deuxième famille préférée de dérivés de 1,3,5-triazine selon l'invention est celle, notamment décrite dans le document EP-A-0570838, des 1,3,5-triazines répondant à la formule (I) et présentant l'ensemble des caractéristiques suivantes :
- X₃ est le radical -NH- ;
- R₃ est choisi parmi : un radical alkyle linéaire ou ramifié en C₁-C₁₈ ; un radical cycloalkyle en C₅-C₁₂ éventuellement substitué avec un ou plusieurs radicaux alkyles en C₁-C₄ ;
- R₁ est choisi parmi : l'hydrogène; un métal alcalin; un radical ammonium; un radical de formule (IV) ; un radical alkyle linéaire ou ramifié en C₁-C₁₈; un radical cycloalkyle en C₅-C₁₂ éventuellement substitué avec un ou plusieurs radicaux alkyles en C₁-C₄ ;
- si X₂ est le radical -NH-, alors R₂ est choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄ ;
- si X₂ est l'oxygène, alors R₂ est choisi parmi l'hydrogène; un métal alcalin; un radical ammonium; un radical de formule (IV); un radical alkyle linéaire ou ramifié en C₁-C₁₈ ; un radical cycloalkyle en C₅-C₁₂ éventuellement substitué avec un ou plusieurs radicaux alkyles en C₁-C₄.

Une 1,3,5-triazine particulièrement préférée de cette deuxième famille est celle répondant à la formule suivante : 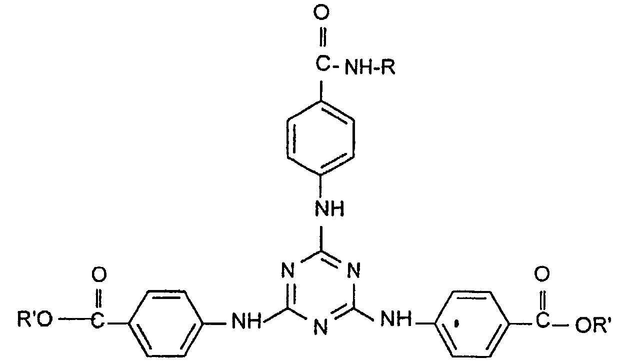 dans laquelle R' désigne un radical éthyl-2 hexyle et R désigne un radical tert. butyle.

Une troisième famille préférée de composés est celle, notamment décrite dans le document US 4,724,137, des 1,3,5-triazines répondant à la formule (I) et présentant l'ensemble des caractéristiques suivantes :
- X₂ et X₃ sont identiques et représentent l'oxygène;
- R₁, R₂ et R₃ sont identiques et représentent un radical alkyle en C₆-C₁₂ ou un radical polyoxyéthyléné comprenant de 1 à 6 unités d'oxyde d'éthylène et dont le groupe OH terminal est méthylé.

Une 1,3,5-triazine particulièrement préférée de cette troisième famille est la 2,4,6-tris[p-(2'-éthylhexyl-1'-oxycarbonyl)anilino]-1,3,5-triazine qui est un filtre connu en soi, actif dans l'UV-B, se présentant sous une forme solide, et qui est vendu notamment sous la dénomination commerciale de "UVINUL T 150" par la Société BASF. Ce produit répond à la formule suivante: 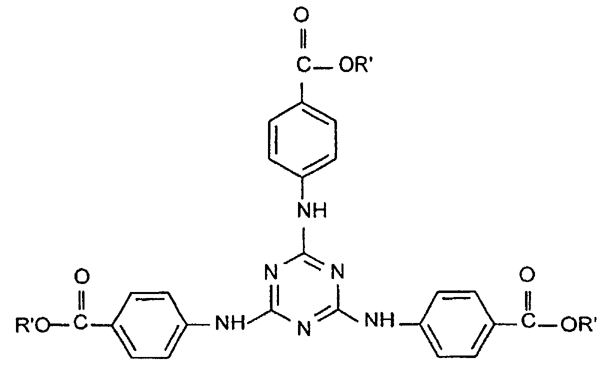 dans laquelle R' désigne un radical 2-éthyl hexyle.

Le ou les dérivés de 1,3,5-triazine sont généralement présents dans les compositions de l'invention à une teneur pouvant aller de 0,5 % à 20 %, de préférence de 1 % à 10 % en poids, par rapport au poids total de la composition.

Comme indiqué précédemment, les dérivés du dibenzoylméthane utilisables selon la présente invention sont ceux répondant à la formule (V) suivante : 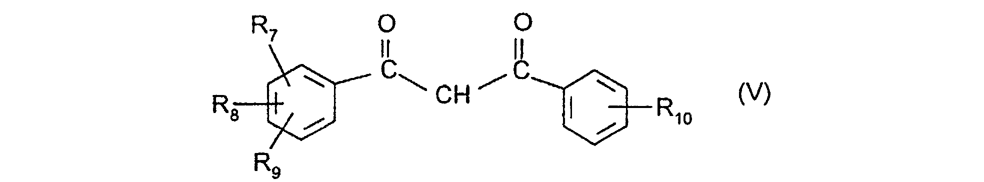 dans laquelle R₇, R₈, R₉ et R₁₀, identiques ou différents, représentent indépendamment l'hydrogène ou un radical hydroxyle ou un radical alkyle linéaire ou ramifié en C₁-C₈ ou un radical alcoxy linéaire ou ramifié en C₁-C₈.

Selon la présente invention, on peut bien entendu mettre en oeuvre un ou plusieurs dérivés du dibenzoylméthane.

Parmi les dérivés du dibenzoylméthane utilisables selon la présente invention, on peut notamment citer, de manière non limitative :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert.-butyl-4'-méthoxydibenzoylméthane,
- le 4,4'-diméthoxydibenzoylméthane.

Ces produits sont déjà bien connus en soi et sont décrits notamment dans les documents FR-A- 2 326 405, FR-A- 2 440 933 et EP-A- 0 114 607 précités.

Parmi les dérivés du dibenzoylméthane mentionnés ci-dessus, on préfère tout particulièrement, selon la présente invention, mettre en oeuvre le 4-(ter.-butyl) 4'-méthoxy dibenzoylméthane, notamment celui proposé à la vente sous la dénomination commerciale de "PARSOL 1789" par la Société GIVAUDAN, ce filtre répondant donc à la formule développée suivante :

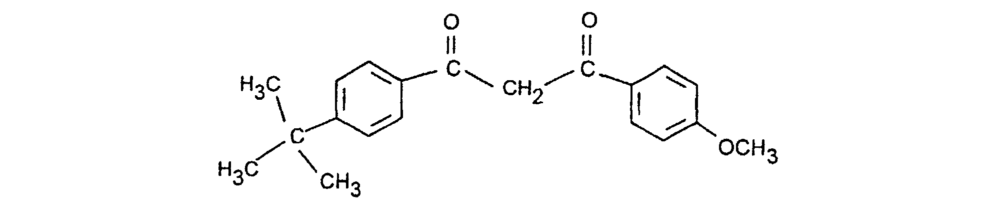

Un autre dérivé du dibenzoylméthane préféré selon la présente invention est le 4-isopropyl-dibenzoylméthane, filtre vendu sous la dénomination de "EUSOLEX 8020" par la Société MERCK, et répondant à la formule développée suivante :

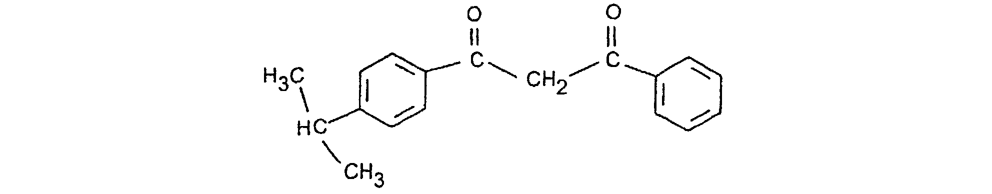

Les dérivés de dibenzoylméthane peuvent être présents dans les compositions de l'invention à une teneur allant de 0,2 % à 15 % en poids, par rapport au poids total de la composition. De préférence, cette teneur va de 0,2 % à 10 %.

Un troisième composé absolument essentiel des compositions selon l'invention est un composé de la famille des composés amidés.

Au sens de la présente invention, on entend par composé amidé tout composé présentant dans sa structure chimique au moins un groupement (ou fonction) amide

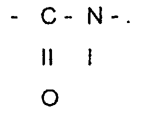

Dans une forme préférée de réalisation de l'invention, les composés amidés répondent à la formule (VI) suivante : 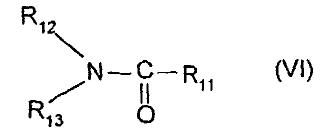 dans laquelle les radicaux R₁₁, R₁₂ et R₁₃, qui peuvent être identiques ou différents, représentent indépendamment l'hydrogène ou des radicaux hydrocarbonés monovalents, saturés ou insaturés, aliphatiques, cycloaliphatiques ou cycliques, éventuellement fonctionnalisés, contenant de 1 à 30 atomes de carbone, de préférence de 1 à 22 atomes de carbone, bornes incluses, étant entendu que, dans cette formule, le radical R₁₁ peut former avec le radical R₁₂ ou avec le radical R₁₃ un cycle contenant inclusivement de 5 à 18 atomes de carbone, et que les radicaux R₁₂ et R₁₃ peuvent ensemble former un cycle contenant de 5 à 18 atomes de carbone, bornes incluses.

Comme exemples de radicaux hydrocarbonés saturés aliphatiques, on peut notamment citer les radicaux alkyle, linéaires ou ramifiés, substitués ou non, en C₁-C₃₀, de préférence en C₁-C₂₂, et en particulier les radicaux méthyle, éthyle, n-propyle, isopropyle, n-butyle, isobutyle, ter.-butyle, pentyle, n-amyle, isoamyle, néopentyle, n-hexyle, n-heptyle, n-octyle, 2-éthylhexyle, ter.-octyle, décyle, lauryle et octadécyle.

A titre d'exemples de radicaux hydrocarbonés saturés cycliques, on peut notamment citer les radicaux cyclopentyle et cyclohexyle, éventuellement substitués, en particulier par des radicaux alkyle.

Comme exemples de radicaux hydrocarbonés insaturés aliphatiques, on peut notamment citer les radicaux alcényle ou alcynyle, linéaires ou ramifiés, substitués ou non, en C₂-C₃₀, de préférence en C₂-C₂₂, et particulier les radicaux vinyle, allyle, oléyle et linoléyle.

Comme exemples de radicaux hydrocarbonés insaturés cycliques, on peut notamment citer les radicaux aryle tels que phényle et naphtyle, éventuellement substitués, en particulier par des alkyle, comme par exemple le radical tolyle, et à titre d'exemples de radicaux cycloaliphatiques insaturés, on peut citer plus particulièrement les radicaux benzyle et phényléthyle.

Par radicaux fonctionnalisés, on entend plus particulièrement des radicaux comportant dans leur structure chimique, tant dans la chaîne principale que sur un chaînon secondaire, un ou plusieurs groupements fonctionnels du type notamment esters, éthers, alcools, amines, amides et cétones, mais de préférence esters.

Parmi les composés amidés de formule (VI) convenant bien à la présente invention, on préfère plus particulièrement mettre en oeuvre les composés présentant au moins l'une, et encore plus préférentiellement l'ensemble, des caractéristiques suivantes :
- le composé amidé est un amide N-substitué, et encore plus préférentiellement N,N-disubstitué,
- R₁₁ est un radical alkyle, linéaire ou ramifié, de préférence en C₁-C₂₂ et encore plus préférentiellement en C₁-C₁₂, ou bien encore un radical phényle lui-même éventuellement substitué par un ou plusieurs radicaux alkyles, linéaires ou ramifiés, en C₁-C₁₂,
- R₁₂ est un radical alkyle, linéaire ou ramifié, de préférence en C₁-C₂₂ et encore plus préférentiellement en C₁-C₁₂,
- R₁₃ est un radical alkyle, linéaire ou ramifié, choisi au sein de ceux définis pour R₁₂, ou bien encore représente un radical monovalent à fonction ester répondant à la formule (VII) suivante : 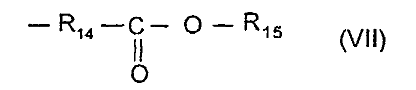 dans laquelle R₁₄ et R₁₅, qui peuvent être identiques ou différents, représentent deux radicaux hydrocarbonés, de préférence de type alkyle, contenant de 1 à 12 atomes de carbone, de préférence de 1 à 8 atomes de carbone.

Selon un mode particulier de réalisation de la présente invention, les composés amidés précédemment définis que l'on met en oeuvre sont des corps gras liquides à température ordinaire. De préférence, ce sont des huiles qui présentent par ailleurs une bonne solubilité dans les phases grasses habituellement utilisées pour la préparation de supports cosmétiquement acceptables.

A titre d'exemples d'huiles amidées spécifiques qui se sont avérées présenter des propriétés absolument remarquables dans la photostabilisation des dérivés de 1,3,5-triazine, en particulier de la 2,4,6-tris[p-(2'-éthylhexyl-1'-oxycarbonyl)anilino]-1,3,5-triazine, on peut plus particulièrement citer :
- les N,N-diéthyl-méthylbenzamides de formule (1) suivante : 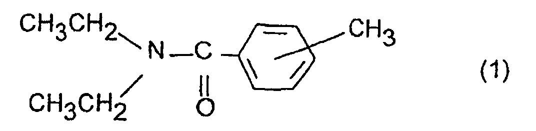 dont le N,N-diéthyl-3-méthylbenzamide,
- le N-butyl, N-acétyl aminopropionate d'éthyle de formule (2) suivante :

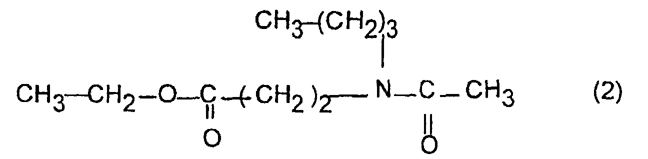

D'une manière générale, le ou les composés amidés peuvent ainsi être présents dans les compositions conformes à l'invention à des teneurs qui sont généralement comprises entre 0,01 % et 50 % en poids, et de préférence à des teneurs comprises entre 0,1 % et 30 % en poids, par rapport au poids total de la composition.

Ainsi, lorsqu'on ajoute en quantité suffisante un composé amidé à une composition antisolaire contenant un dérivé de dibenzoylméthane, en particulier du 4-tert-butyl-4'-méthoxydibenzoylméthane, et un dérivé de 1,3,5-triazine tel que défini ci-dessus, on observe une augmentation de la stabilité dudit dérivé de 1,3,5-triazine à la lumière, et donc une amélioration de l'efficacité de la composition antisolaire au cours du temps.

Les compositions cosmétiques et/ou dermatologiques visées par la présente invention peuvent bien entendu contenir un ou plusieurs filtres solaires complémentaires actifs dans l'UVA et/ou l'UVB (absorbeurs), hydrophiles ou lipophiles, autres bien sûr que les trois filtres mentionnés ci-avant. Ces filtres complémentaires peuvent être notamment choisis parmi les dérivés cinnamiques, les dérivés salicyliques, les dérivés du benzylidène camphre, les dérivés de benzimidazole, les dérivés de triazine autres que ceux ci-avant mentionnés, les dérivés de la benzophénone, les dérivés de β,β'-diphénylacrylate, les dérivés de l'acide p-aminobenzoïque, les polymères filtres et silicones filtres décrits dans la demande WO-93/04665. D'autres exemples de filtres organiques sont donnés dans la demande de brevet EP-A 0 487 404.

Les compositions selon l'invention peuvent également contenir des agents de bronzage et/ou de brunissage artificiels de la peau (agents autobronzants), tels que par exemple de la dihydroxyacétone (DHA).

Les compositions cosmétiques et/ou dermatologiques selon l'invention peuvent encore contenir des pigments ou bien encore des nanopigments (taille moyenne des particules primaires : généralement entre 5 nm et 100 nm, de préférence entre 10 et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs bien connus en soi agissant par blocage physique (réflexion et/ou diffusion) du rayonnement UV. Des agents d'enrobage classiques sont par ailleurs l'alumine et/ou le stéarate d'aluminium. De tels nanopigments d'oxydes métalliques, enrobés ou non enrobés, sont en particulier décrits dans les demande de brevets EP-A- 0 518 772 et EP-A- 0 518 773.

Les compositions conformes à la présente invention peuvent comprendre en outre des adjuvants cosmétiques classiques notamment choisis parmi les corps gras, les solvants organiques, les épaississants ioniques ou non ioniques, les adoucissants, les antioxydants, les agents anti radicaux libres, les opacifiants, les stabilisants, les émollients, les silicones , les α-hydroxyacides, les agents anti-mousse, les agents hydratants, les vitamines, les parfums, les conservateurs, les tensioactifs, les charges, les séquestrants, les polymères, les propulseurs, les agents alcalinisants ou acidifiants, les colorants, ou tout autre ingrédient habituellement utilisé dans le domaine cosmétique et/ou dermatologique, en particulier pour la fabrication de compositions antisolaires sous forme d'émulsions.

Les corps gras peuvent être constitués par une huile ou une cire ou leurs mélanges. Par huile, on entend un composé liquide à température ambiante. Par cire, on entend un composé solide ou substantiellement solide à température ambiante, et dont le point de fusion est généralement supérieur à 35°C.

Comme huiles, on peut citer les huiles minérales (vaseline); végétales (huile d'amande douce, de macadamia, de pépin de cassis, de jojoba) ; synthétiques comme le perhydrosqualène, les alcools, les acides ou les esters gras (comme le benzoate d'alcools en C₁₂-C₁₅ vendu sous la dénomination commerciale « Finsolv TN » par la société Finetex, le palmitate d'octyle, le lanolate d'isopropyle, les triglycérides dont ceux des acides caprique/caprylique), les esters et éthers gras oxyéthylénés ou oxypropylénés; siliconées (cyclométhicone, polydiméthysiloxanes ou PDMS) ou fluorées, les polyalkylènes.

Comme composés cireux, on peut citer la paraffine, la cire de carnauba, la cire d'abeille, l'huile de ricin hydrogénée.

Parmi les solvants organiques, on peut citer les alcools et polyols inférieurs.

Les épaississants peuvent être choisis notamment parmi les acides polyacryliques réticulés, les gommes de guar et celluloses modifiées ou non telles que la gomme de guar hydroxypropylée, la méthylhydroxyéthylcellulose et l'hydroxypropylméthyl cellulose.

Bien entendu, l'homme de l'art veillera à choisir le ou les éventuels composés complémentaires cités ci-dessus (en particulier les filtres complémentaires) et/ou leurs quantités de manière telle que les propriétés avantageuses attachées intrinsèquement à l'association ternaire conforme à l'invention ne soient pas, ou substantiellement pas, altérées par la ou les adjonctions envisagées et de manière telle que les compositions de l'invention présentent de bonnes propriétés cosmétiques.

Les compositions selon l'invention peuvent être préparées selon les techniques bien connues de l'homme de l'art, en particulier celles destinées à la préparation d'émulsions de type huile-dans-eau ou eau-dans-huile.

Cette composition peut se présenter en particulier sous forme d'émulsion, simple ou complexe (H/E, E/H, H/E/H ou E/H/E) telle qu'une crème, un lait, ou sous la forme d'un gel ou d'un gel crème, de poudre, de bâtonnet solide et éventuellement être conditionnée en aérosol et se présenter sous forme de mousse ou de spray.

De préférence, les compositions selon l'invention se présentent sous la forme d'une émulsion huile-dans-eau.

Lorsqu'il s'agit d'une émulsion, la phase aqueuse de celle-ci peut comprendre une dispersion vésiculaire non ionique préparée selon des procédés connus (Bangham, Standish and Watkins. J. Mol. Biol. 13, 238 (1965), FR 2 315 991 et FR 2 416 008).

La composition cosmétique et/ou dermatologique de l'invention peut être utilisée comme composition protectrice de l'épiderme humain ou des cheveux contre les rayons ultraviolets, comme composition antisolaire ou comme produit de maquillage.

Lorsque la composition cosmétique selon l'invention est utilisée pour la protection de l'épiderme humain contre les rayons UV, ou comme composition antisolaire, elle peut se présenter sous forme de suspension ou de dispersion dans des solvants ou des corps gras, sous forme de dispersion vésiculaire non ionique ou encore sous forme d'émulsion, de préférence de type huile-dans-eau, telle qu'une crème ou un lait, sous forme de pommade, de gel, de gel crème, de bâtonnet solide, de stick, de mousse aérosol ou de spray.

Lorsque la composition cosmétique selon l'invention est utilisée pour la protection des cheveux, elle peut se présenter sous forme de shampooing, de lotion, de gel, d'émulsion, de dispersion vésiculaire non ionique, de laque pour cheveux et constituer par exemple une composition à rincer, à appliquer avant ou après shampooing, avant ou après coloration ou décoloration, avant, pendant ou après permanente ou défrisage, une lotion ou un gel coiffants ou traitants, une lotion ou un gel pour le brushing ou la mise en plis, une composition de permanente ou de défrisage, de coloration ou décoloration des cheveux.

Lorsque la composition est utilisée comme produit de maquillage des cils, des sourcils ou de la peau, tel que crème de traitement de l'épiderme, fond de teint, bâton de rouge à lèvres, fard à paupières, fard à joues, mascara ou ligneur encore appelé "eye liner", elle peut se présenter sous forme solide ou pâteuse, anhydre ou aqueuse, comme des émulsions huile dans eau ou eau dans huile, des dispersions vésiculaires non ioniques ou encore des suspensions.

A titre indicatif, pour les formulations antisolaires conformes à l'invention qui présentent un support de type émulsion huile-dans-eau, la phase aqueuse (comprenant notamment les filtres hydrophiles) représente généralement de 50 à 95 % en poids, de préférence de 70 à 90 % en poids, par rapport à l'ensemble de la formulation, la phase huileuse (comprenant notamment les filtres lipophiles) de 5 à 50 % en poids, de préférence de 10 à 30 % en poids, par rapport à l'ensemble de la formulation, et le ou les (co)émulsionnant(s) de 0,5 à 20 % en poids, de préférence de 2 à 10% en poids, par rapport à l'ensemble de la formulation.

Un exemple concret, mais nullement limitatif, illustrant l'invention, va maintenant être donné.

### EXEMPLE :

On a réalisé trois émulsions huile-dans-eau A, B et C dont le support commun présente la composition suivante (les quantités sont exprimées en % de poids par rapport au poids total de la composition) :
- mélange mono-stéarate de glycérol/stéarate de polyéthylène glycol (100 OE) vendu sous la dénomination commerciale « ARLACEL 165 » par ICI 2 %
- benzoate d'alcools C12/C15 vendu sous la dénomination commerciale « FINSOLV TN » par Finetex 15 %
- acide éthylène diamine tétracétique, sel disodique, 2 H₂0 0,1 %
- glycérine 3 %
- sorbitol 2 %
- copolymère acide acrylique/acétate d'alkyle (C10-C30) réticulé vendu sous la dénomination commerciale « PEMULEN TR1 » par Goodrich 0,5 %
- triéthanolamine 0,5 %
- conservateurs qs
- eau déminéralisée qsp 100%

L'émulsion A (comparative) comprend en outre un dérivé de 1,3,5 triazine qui est la 2,4,6-tris[p-(2'-éthylhexyl-1'-oxycarbonyl)anilino]-1,3,5-triazine (UVINUL T150). L'émulsion B, également comparative, contient de l'UVINUL T150 en association avec du 4-tert-butyl-4'-méthoxydibenzoylméthane (PARSOL 1789). L'émulsion C, selon l'invention, comprend, outre de l'UVINUL T150 et du PARSOL 1789, du N-butyl N-acétyl aminopropionate d'éthyle vendu sous la dénomination commerciale « R3535 » par Merck.

Les compositions des émulsions A, B et C au niveau des différents composés cités ci-dessus qu'elles contiennent sont rassemblées dans le tableau (I) ci-dessous (les quantités sont exprimées en % de poids par rapport au poids total de la composition) :

**Tableau (I) :**

| Composé | Emulsion A (comparative) | Emulsion B (comparative) | Emulsion C (invention) |
|---|---|---|---|
| Uvinul T 150 | 1,5 % | 1,5 % | 1,5% |
| Parsol 1789 | - | 0,5 % | 0,5% |
| R3535 | - | - | 15% |

Pour chacune de ces émulsions, on a déterminé le pourcentage de 2,4,6-tris[p-(2'-éthylhexyl-1'-oxycarbonyl)anilino]-1,3,5-triazine résiduelle après irradiation par des UV selon le protocole suivant : pour chaque formule, on a préparé quatre échantillons témoins et quatre échantillons tests. On a déposé sur des plaques de PMMA (polyméthacrylate de méthyle) dépolies, préalablement rincées à l'eau puis séchées, 16 mg de formule qu'on a étalée sur une surface de 2 cm x 4 cm. Puis on a irradié les plaques (SUNTEST CPS Heraeus) pendant 4 heures dans une enceinte dont la température est régulée aux environs de 35-40 °C afin de simuler une irradiation UV naturelle en conservant les plaques témoins à l'obscurité pendant le temps d'irradiation des autres plaques.

On a ensuite dosé les échantillons de la manière suivante : on a procédé à l'extraction des filtres en immergeant chaque plaque dans 55 ml d'éthanol afin de solubiliser les filtres. Les plaques et le solvant contenant les filtres ont ensuite été traités aux ultrasons pendant 5 minutes pour assurer une extraction efficace. Les solutions obtenues sont analysées par chromatographie en phase liquide haute performance.

Pour chaque formule testée, le taux de 2,4,6-tris[p-(2'-éthylhexyl-1'-oxycarbonyl)anilino]-1,3,5-triazine résiduelle après irradiation est donné par le rapport de sa concentration dans l'échantillon irradié à sa concentration dans l'échantillon non irradié.

Les résultats en pourcentage de 2,4,6-tris[p-(2'-éthylhexyl-1'-oxycarbonyl)anilino]-1,3,5-triazine restante sont consignés dans le tableau (II) suivant :

**Tableau (II) :**

| Emulsion | Uvinul T 150 résiduel |
|---|---|
| Emulsion A (comparative) | 93 % |
| Emulsion B (comparative) | 69 % |
| Emulsion C (invention) | 87 % |

## Revendications

1. Composition cosmétique et/ou dermatologique comprenant, dans un support cosmétiquement et/ou dermatologiquement acceptable :
- i) au moins un dérivé de 1,3,5-triazine répondant à la formule (I) suivante : 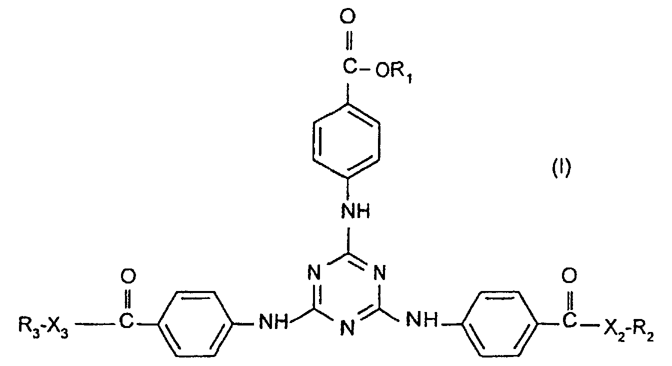 dans laquelle :
- X₂ et X₃, identiques ou différents, représentent l'oxygène ou le radical -NH- ;
- R₁, R₂ et R₃, identiques ou différents, sont choisis parmi : l'hydrogène; un métal alcalin ; un radical ammonium éventuellement substitué par un ou plusieurs radicaux alkyles ou hydroxyalkyles; un radical alkyle linéaire ou ramifié en C₁-C₁₈ ; un radical cycloalkyle en C₅-C₁₂ éventuellement substitué par un ou plusieurs radicaux alkyles en C₁-C₄ ; un radical polyoxyéthyléné comprenant de 1 à 6 unités d'oxyde d'éthylène et dont le groupe OH terminal est méthylé; un radical de formule (Il), (III) ou (IV) suivantes : 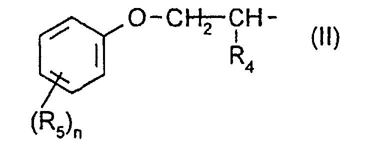 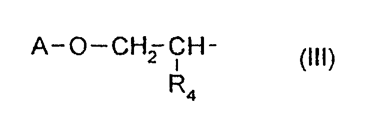 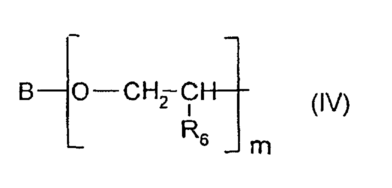
dans lesquelles :
- R₄ est l'hydrogène ou un radical méthyle ;
- R₅ est un radical alkyle en C₁-C₉ ;
- n est un nombre entier allant de 0 à 3 ;
- m est un nombre entier allant de 1 à 10 ;
- A est un radical alkyle en C₄-C₈ ou un radical cycloalkyle en C₅-C₈ ;
- B est choisi parmi : un radical alkyle linéaire ou ramifié en C₁-C₈ ; un radical cycloalkyle en C₅-C₈ ; un radical aryle éventuellement substitué par un ou plusieurs radicaux alkyles en C₁-C₄ ;
- R₆ est l'hydrogène ou un radical méthyle,
- ii) au moins un dérivé de dibenzoylméthane répondant à la formule (V) suivante : 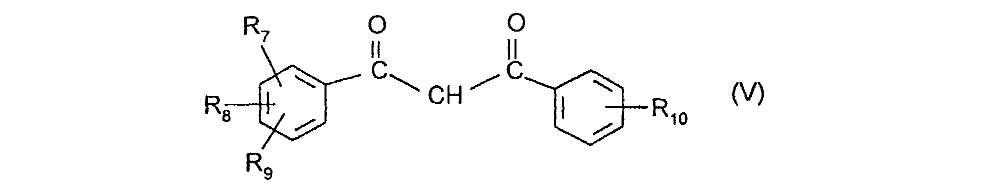 dans laquelle R₇, R₈, R₉ et R₁₀, identiques ou différents, représentent indépendamment l'hydrogène ou un radical hydroxyle ou un radical alkyle linéaire ou ramifié en C₁-C₈ ou un radical alcoxy linéaire ou ramifié en C₁-C₈,
- et iii) au moins un composé amidé.

2. Composition selon la revendication 1, **caractérisée par le fait que** le dérivé de 1,3,5-triazine est choisi parmi ceux de formule (I) présentant l'ensemble des caractéristiques suivantes :
- X₂ et X₃ sont identiques et représentent l'oxygène ;
- R₁ est choisi parmi : un radical cycloalkyle en C₅-C₁₂ éventuellement substitué par un ou plusieurs radicaux alkyles en C₁-C₄ ; un radical de formule (II), (III) ou (IV) dans lesquelles :
- B est un radical alkyle en C₁-C₄;
- R₆ est le radical méthyle;
- R₂ et R₃, identiques ou différents, sont choisis parmi : l'hydrogène; un métal alcalin; un radical ammonium éventuellement substitué par un ou plusieurs radicaux alkyles ou hydroxyalkyles; un radical alkyle linéaire ou ramifié en C₁-C₁₈; un radical cycloalkyle en C₅-C₁₂ éventuellement substitué par un ou plusieurs radicaux alkyles en C₁-C₄; un radical de formule (Il), (III) ou (IV) dans lesquelles :
- B est un radical alkyle en C₁-C₄;
- R₆ est le radical méthyle.

3. Composition selon la revendication 1, **caractérisée par le fait que** le dérivé de 1,3,5-triazine est choisi parmi ceux de formule (I) présentant l'ensemble des caractéristiques suivantes :
- X₂ et X₃ sont identiques et représentent le radical -NH- ;
- R₃ est choisi parmi : un radical alkyle linéaire ou ramifié en C₁-C₁₈ ; un radical cycloalkyle en C₅-C₁₂ éventuellement substitué avec un ou plusieurs radicaux alkyles en C₁-C₄ ;
- R₁ est choisi parmi : l'hydrogène; un métal alcalin; un radical ammonium; un radical de formule (IV); un radical alkyle linéaire ou ramifié en C₁-C₁₈; un radical cycloalkyle en C₅-C₁₂ éventuellement substitué avec un ou plusieurs radicaux alkyles en C₁-C₄ ;
- R₂ est choisi parmi : un radical alkyle linéaire ou ramifié en C₁-C₁₈ ; un radical cycloalkyle en C₅-C₁₂ éventuellement substitué avec un ou plusieurs radicaux alkyles en C₁-C₄.

4. Composition selon la revendication 1, **caractérisée par le fait que** le dérivé de 1,3,5-triazine est choisi parmi ceux présentant l'ensemble des caractéristiques suivantes :
- X₂ est l'oxygène;
- X₃ est le radical -NH-;
- R₃ est choisi parmi : un radical alkyle linéaire ou ramifié en C₁-C₁₈; un radical cycloalkyle en C₅-C₁₂ éventuellement substitué avec un ou plusieurs radicaux alkyles en C₁-C₄;
- R₁ est choisi parmi : l'hydrogène; un métal alcalin; un radical ammonium; un radical de formule (IV); un radical alkyle linéaire ou ramifié en C₁-C₁₈; un radical cycloalkyle en C₅-C₁₂ éventuellement substitué avec un ou plusieurs radicaux alkyles en C₁-C₄;
- R₂ est choisi parmi l'hydrogène; un métal alcalin; un radical ammonium; un radical de formule (IV); un radical alkyle linéaire ou ramifié en C₁-C₁₈; un radical cycloalkyle en C₅-C₁₂ éventuellement substitué avec un ou plusieurs radicaux alkyles en C₁-C₄.

5. Composition selon la revendication 4, **caractérisée par le fait que** le dérivé de 1,3,5-triazine est celui répondant à la formule suivante : 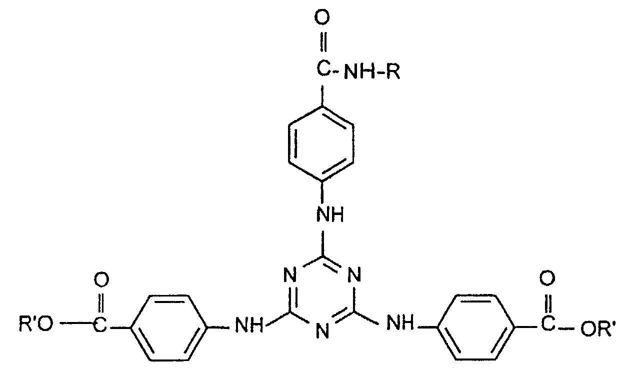 dans laquelle R' désigne un radical éthyl-2 hexyle et R désigne un radical ter. butyle.

6. Composition selon la revendication 1, **caractérisée par le fait que** le dérivé de 1,3,5-triazine est choisi parmi ceux présentant l'ensemble des caractéristiques suivantes :
- X₂ et X₃ sont identiques et représentent l'oxygène;
- R₁, R₂ et R₃ sont identiques et représentent un radical alkyle en C₆-C₁₂ ou un radical polyoxyéthyléné comprenant de 1 à 6 unités d'oxyde d'éthylène et dont le groupe OH terminal est méthylé.

7. Composition selon la revendication 6, **caractérisée par le fait que** le dérivé de 1,3,5-triazine est celui répondant à la formule suivante : 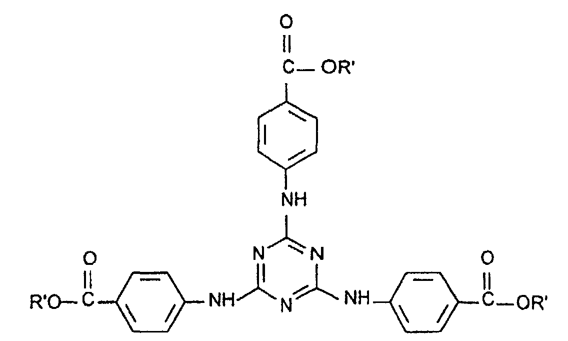 dans laquelle R' désigne un radical 2-éthyl hexyle.

8. Composition selon l'une quelconque des revendications 1 à 7, **caractérisée par le fait que** le dérivé de 1,3,5-triazine est présent dans la composition à une teneur allant de 0,5 % à 20 % en poids, par rapport au poids total de la composition, de préférence de 1 % à 10 % en poids, par rapport au poids total de la composition.

9. Composition selon l'une quelconque des revendications 1 à 8, **caractérisée par le fait que** dérivé de dibenzoylméthane est choisi parmi :
- le 2-méthyldibenzoylméthane,
- le 4-méthyldibenzoylméthane,
- le 4-isopropyldibenzoylméthane,
- le 4-tert.-butyldibenzoylméthane,
- le 2,4-diméthyldibenzoylméthane,
- le 2,5-diméthyldibenzoylméthane,
- le 4,4'-diisopropyldibenzoylméthane,
- le 4-tert.-butyl-4'-méthoxydibenzoylméthane,
- le 2-méthyl-5-isopropyl-4'-méthoxydibenzoylméthane,
- le 2-méthyl-5-tert.-butyl-4'-méthoxydibenzoylméthane,
- le 2,4-diméthyl-4'-méthoxydibenzoylméthane,
- le 2,6-diméthyl-4-tert.-butyl-4'-méthoxydibenzoylméthane,
- le 4,4'-diméthoxydibenzoylméthane.

10. Composition selon l'une quelconque des revendications précédentes, **caractérisée par le fait que** le dérivé de dibenzoylméthane est le 4-tert.-butyl-4'-méthoxydibenzoylméthane.

11. Composition selon l'une quelconque des revendications précédentes, **caractérisée par le fait que** le dérivé de dibenzoylméthane est présent dans la composition à une teneur allant de 0,2 % à 15 % en poids, par rapport au poids total de la composition, de préférence, de 0,2 % à 10 % en poids, par rapport au poids total de la composition.

12. Composition selon l'une quelconque des revendications précédentes, **caractérisée par le fait que** le composé amidé est choisi parmi ceux répondant à la formule (VI) suivante : 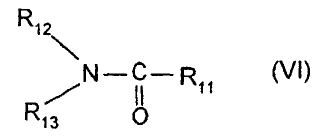 dans laquelle les radicaux R₁₁, R₁₂ et R₁₃, qui peuvent être identiques ou différents, représentent indépendamment l'hydrogène ou des radicaux hydrocarbonés monovalents, saturés ou insaturés, aliphatiques, cycloaliphatiques ou cycliques, éventuellement fonctionnalisés, contenant de 1 à 30 atomes de carbone, de préférence de 1 à 22 atomes de carbone, bornes incluses, étant entendu que, dans cette formule, le radical R₁₁ peut former avec le radical R₁₂ ou avec le radical R₁₃ un cycle contenant inclusivement de 5 à 18 atomes de carbone, et que les radicaux R₁₂ et R₁₃ peuvent ensemble former un cycle contenant de 5 à 18 atomes de carbone, bornes incluses.

13. Composition selon la revendication 12, **caractérisée par le fait que** le composé amidé est choisi parmi ceux présentant au moins l'une, et encore plus préférentiellement l'ensemble, des caractéristiques suivantes :
- le composé amidé est un amide N-substitué, et encore plus préférentiellement N,N-disubstitué,
- R₁₁ est un radical alkyle, linéaire ou ramifié, de préférence en C₁-C₂₂ et encore plus préférentiellement en C₁-C₁₂, ou bien encore un radical phényle lui-même éventuellement substitué par un ou plusieurs radicaux alkyles, linéaires ou ramifiés, en C₁-C₁₂,
- R₁₂ est un radical alkyle, linéaire ou ramifié, de préférence en C₁-C₂₂ et encore plus préférentiellement en C₁-C₁₂,
- R₁₃ est un radical alkyle, linéaire ou ramifié, choisi au sein de ceux définis pour R₁₂, ou bien encore représente un radical monovalent à fonction ester répondant à la formule (VII) suivante : 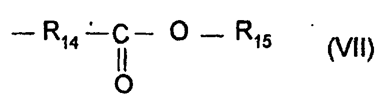
dans laquelle R₁₄ et R₁₅, qui peuvent être identiques ou différents, représentent deux radicaux hydrocarbonés, de préférence de type alkyle, contenant de 1 à 12 atomes de carbone, de préférence de 1 à 8 atomes de carbone.

14. Composition selon l'une quelconque des revendications précédentes, **caractérisée par le fait que** le composé amidé est le le N-butyl, N-acétyl aminopropionate d'éthyle.

15. Composition selon l'une quelconque des revendications précédentes, **caractérisée par le fait que** le composé amidé est présent dans la composition à une teneur allant de 0,01 % à 50 % en poids, par rapport au poids total de la composition, de préférence de 0,1 % à 30 % en poids, par rapport au poids total de la composition.

16. Composition selon l'une quelconque des revendications précédentes, **caractérisée par le fait qu'**elle est sous la forme d'une émulsion huile-dans-eau.

17. Utilisation d'un composé amidé tel que défini à l'une quelconque des revendications 1, 12, 13 ou 14 dans des compositions cosmétiques et/ou dermatologiques contenant un dérivé de dibenzoylméthane tel que défini à l'une quelconque des revendications 1, 9 ou 10 en association avec au moins un-dérivé de 1,3,5-triazine tel que défini à l'une quelconque des revendications 1 à 7 en vue d'améliorer dans lesdites compositions la stabilité au rayonnement UV dudit dérivé de 1,3,5-triazine.

18. Procédé pour améliorer la stabilité au rayonnement UV des compositions cosmétiques et/ou dermatologiques comprenant un dérivé de dibenzoylméthane tel que défini à l'une quelconque des revendications 1, 9 ou 10 et un dérivé de 1,3,5-triazine tel que défini à l'une quelconque des revendications 1 à 7, **caractérisé par le fait qu'**il consiste à introduire dans lesdites compositions une quantité efficace d'un composé amidé tel que défini à l'une quelconque des revendications 1, 12, 13 ou 14.

## Patentansprüche

1. Kosmetische und/oder dermatologische Zusammensetzung, die in einem kosmetisch und/oder dermatologisch akzeptablen Träger enthält:
i) mindestens ein 1,3,5-Triazinderivat der folgenden Formel (I): 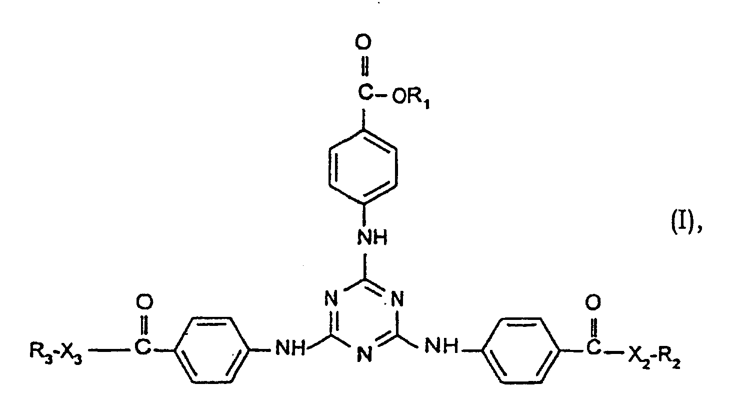 worin:
- die Gruppen X₂ und X₃, die identisch oder voneinander verschieden sind, Sauerstoff oder die Gruppe -NH- bedeuten; und
- die Gruppen R₁, R₂ und R₃, die identisch oder voneinander verschieden sind, ausgewählt sind unter: Wasserstoff; einem Alkalimetall; einer Ammoniumgruppe, die gegebenenfalls mit einer oder mehreren Alkyl- oder Hydroxyalkylgruppen substituiert ist; einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist; einer Polyoxyethylengruppe, die 1 bis 6 Ethylenoxideinheiten aufweist und deren endständige OH-Gruppe methyliert ist; einer Gruppe der folgenden Formeln (II), (III) oder (IV): 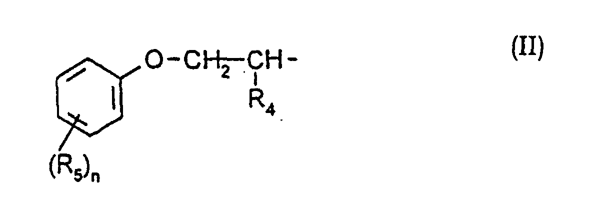 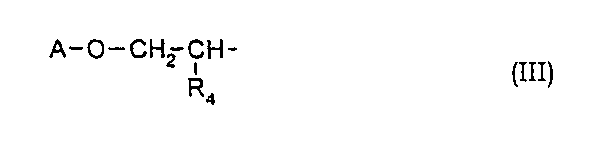 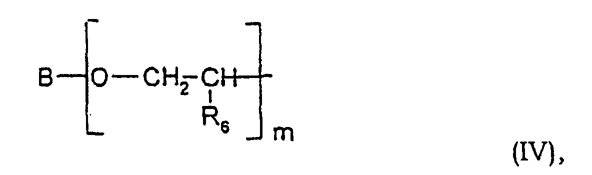
worin bedeuten:
- R₄ Wasserstoff oder Methyl;
- R₅ eine C₁₋₉-Alkylgruppe;
- n Null oder eine ganze Zahl von 1 bis 3;
- m eine ganze Zahl im Bereich von 1 bis 10;
- A eine C₄₋₈-Alkylgruppe oder eine C₅₋₈-Cycloalkylgruppe;
- B eine geradkettige oder verzweigte C₁₋₈-Alkylgruppe; eine C₅₋₈-Cycloalkylgruppe; eine Arylgruppe, die gegebenenfalls mit einer oder mehreren C₁₋₄-Alkylgruppe substituiert ist;
- R₆ Wasserstoff oder Methyl;
ii) mindestens ein Dibenzoylmethanderivat der folgenden Formel (V): 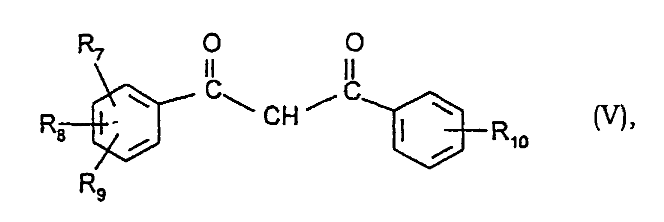 worin die Gruppen R₇, R₈, R₉ und R₁₀, die identisch oder voneinander verschieden sind, unabhängig voneinander Wasserstoff, Hydroxy, eine geradkettige oder verzweigte C₁₋₈-Alkylgruppe oder eine geradkettige oder verzweigte C₁₋₈-Alkoxygruppe bedeuten;
und iii) mindestens eine amidierte Verbindung.

2. Zusammensetzung nach Anspruch 1, **dadurch gekennzeichnet, daß** das 1,3,5-Triazinderivat unter den Derivaten der Formel (I) ausgewählt ist, die die gesamten folgenden Eigenschaften aufweisen:
- X₂ und X₃ sind identisch und bedeuten Sauerstoff;
- R₁ ist ausgewählt unter: einer C₅₋₁₂-Cycloalkylgruppe, die gegebenenfalls mit einer oder mehreren C₁₋₄-Alkylgruppen substituiert ist; einer Gruppe der Formel (II), (III) oder (IV), worin bedeuten:
- B eine C₁₋₄-Alkylgruppe;
- R₆ Methyl;
- R₂ und R₃, die identisch oder voneinander verschieden sind, sind ausgewählt unter: Wasserstoff; einem Alkalimetall; einer Ammoniumgruppe, die gegebenenfalls mit einer oder mehreren Alkyl- oder Hydroxyalkylgruppen substituiert ist; einer geradkettigen oder verzweigten C₁₋₁₈-Alkylgruppe; einer C₅₋₁₂-Cycloalkyl-gruppe, die gegebenenfalls mit einer oder mehreren C₁₋₄-Alkylgruppen substituiert ist; einer Gruppe der Formel (II), (III) oder (IV), worin bedeuten:
- B eine C₁₋₄-Alkylgruppe;
- R₆ Methyl.

3. Zusammensetzung nach Anspruch 1, **dadurch gekennzeichnet, daß** das 1,3,5,-Triazinderivat unter den Derivaten der Formel (I) ausgewählt ist, die die gesamten folgenden Eigenschaften aufweisen:
- X₂ und X₃ sind identisch und bedeuten die Gruppe -NH-;
- R₃ ist ausgewählt unter einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist;
- R₁ ist ausgewählt unter Wasserstoff; einem Alkalimetall; einer Ammoniumgruppe; einer Gruppe der Formel (IV); einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist;
- R₂ ist ausgewählt unter: einer geradkettigen oder verzweigten C₁₋₁₈ -Alkylgruppe; einer C₅₋₁₂-Cycloalkylgruppe, die gegebenenfalls mit einer oder mehreren C₁₋₄-Alkylgruppen substituiert ist.

4. Zusammensetzung nach Anspruch 1, **dadurch gekennzeichnet, daß** das 1,3,5-Triazinderivat unter den Derivaten ausgewählt ist, die die gesamten folgenden Eigenschaften aufweisen:
- X₂ ist Sauerstoff;
- X₃ ist die Gruppe -NH-;
- R₃ ist ausgewählt unter einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist;
- R₁ ist ausgewählt unter Wasserstoff; einem Alkalimetall; einer Ammoniumgruppe; einer Gruppe der Formel (IV); einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist;
- R₂ ist ausgewählt unter Wasserstoff; einem Alkalimetall; einer Ammoniumgruppe; einer Gruppe der Formel (IV); einer geradkettigen oder verzweigten C₁₋₁₈-Alkylgruppe; einer C₅₋₁₂-Cycloalkylgruppe, die gegebenenfalls mit einer oder mehreren C₁₋₄-Alkylgruppen substituiert ist.

5. Zusammensetzung nach Anspruch 4, **dadurch gekennzeichnet, daß** das 1,3,5-Triazinderivat der folgenden Formel entspricht: 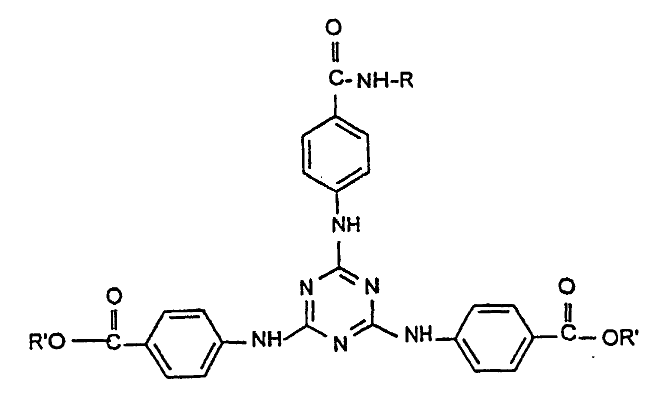 worin die Gruppen R' die 2-Ethylhexylgruppe und R die *t*-Butylgruppe bedeuten.

6. Zusammensetzung nach Anspruch 1, **dadurch gekennzeichnet, daß** das 1,3,5-Triazinderivat unter den Derivaten ausgewählt ist, die alle folgenden Eigenschaften aufweisen:
- X₂ und X₃ sind identisch und bedeuten Sauerstoff;
- R₁, R₂ und R₃ sind identisch und bedeuten eine C₆₋₁₂-Alkylgruppe oder eine Polyoxyethylengruppe mit 1 bis 6 Ethylenoxideinheiten, deren OH-Endgruppe methyliert ist.

7. Zusammensetzung nach Anspruch 6, **dadurch gekennzeichnet, daß** das 1,3,5-Triazinderivat der folgenden Formel entspricht: 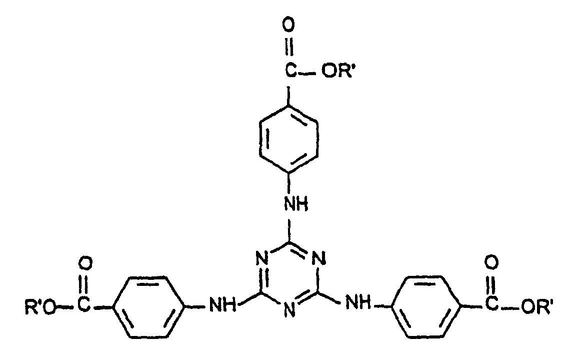 worin die Gruppen R' die 2-Ethylhexylgruppe bedeuten.

8. Zusammensetzung nach einem der Ansprüche 1 bis 7, **dadurch gekennzeichnet, daß** das 1,3,5-Triazinderivat in der Zusammensetzung in einem Mengenanteil von 0,5 bis 20 Gew.-%, bezogen auf das Gesamtgewicht der Zusammensetzung, und vorzugsweise im Bereich von 1 bis 10 Gew.-%, bezogen auf das Gesamtgewicht der Zusammensetzung, vorliegt.

9. Zusammensetzung nach einem der Ansprüche 1 bis 8, **dadurch gekennzeichnet, daß** das Dibenzoylmethanderivat ausgewählt ist unter:
- 2-Methyl-dibenzoylmethan,
- 4-Methyl-dibenzoylmethen,
- 4-Isoproyl-dibenzoylmethan,
- 4-*t*-Butyl-dibenzoylmethan,
- 2,4-Dimethyl-dibenzoylmethan,
- 2,5-Dimethyl-dibenzoylmethan,
- 4,4'-Diisopropyl-dibenzoylmethan,
- 4*-t*-Butyl-4'-methoxy-dibenzoylmethan,
- 2-Methyl-5-isopropyl-4'-methoxy-dibenzoylmethan,
- 2-Methyl-5-*t*-butyl-4'-methoxy-dibenzoylmethan,
- 2,4-Dimethyl-4'-methoxy-dibenzoylmethan,
- 2,6-Dimethyl-4-*t*-butyl-4'-methoxy-dibenzoylmethan, und
- 4,4'-Dimethoxy-dibenzoylmethan.

10. Zusammensetzung nach einem der vorhergehenden Ansprüche, **dadurch gekennzeichnet, daß** das Dibenzoylmethanderivat das 4-*t*-Butyl-4'-methoxydibenzoylmethan ist.

11. Zusammensetzung nach einem der vorhergehenden Ansprüche, **dadurch gekennzeichnet, daß** das Dibenzoylmethanderivat in der Zusammensetzung in einem Mengenanteil von 0,2 bis 15 Gew.-%, bezogen auf das Gesamtgewicht der Zusammensetzung, und vorzugsweise im Bereich von 0,2 bis 10 Gew.-%, bezogen auf das Gesamtgewicht der Zusammensetzung, vorliegt.

12. Zusammensetzung nach einem der vorhergehenden Ansprüche, **dadurch gekennzeichnet, daß** die amidierte Verbindung unter den Verbindungen der folgenden Formel (VI) ausgewählt ist: 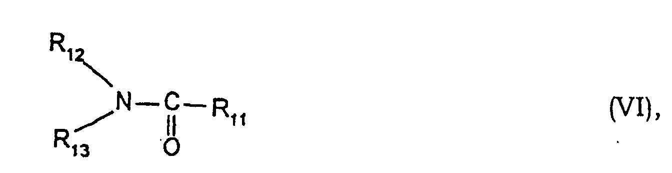 worin die Gruppen R₁₁, R₁₂ und R₁₃, die identisch oder voneinander verschieden sein können, unabhängig voneinander Wasserstoff oder einwertige, gesättigte oder ungesättigte, aliphatische, cycloaliphatische oder cyclische, funktionalisierte Kohlenwasserstoffgruppen bedeuten, die 1 bis 30 Kohlenstoffatome und vorzugsweise 1 bis 22 Kohlenstoffatomen aufweisen (Grenzen eingeschlossen), mit der Maßgabe, daß R₁₁ entweder mit R₁₂ oder mit R₁₃ einen Ring bilden kann, der 5 bis 18 Kohlenstoffatome aufweist, und R₁₂ und R₁₃ gemeinsam einen Ring bilden können, der 5 bis 18 Kohlenstoffatome aufweist.

13. Zusammensetzung nach Anspruch 12, **dadurch gekennzeichnet, daß** die amidierte Verbindung unter den Verbindungen ausgewählt ist, die mindestens eine und vorzugsweise alle folgenden Eigenschaften aufweisen:
- die amidierte Verbindung ist ein N-substituiertes Amid und noch bevorzugter ein N,N-disubstituiertes Amid;
- die Gruppe R₁₁ ist eine geradkettige oder verzweigte Alkylgruppe, die vorzugsweise 1 bis 22 und noch bevorzugter 1 bis 12 Kohlenstoffatome aufweist, oder eine Phenylgruppe, die gegebenenfalls mit einer oder mehreren, geradkettigen oder verzweigten C₁₋₁₂-Alkylgruppen substituiert ist;
- die Gruppe R₁₂ ist eine geradkettige oder verzweigte Alkylgruppe, die vorzugsweise 1 bis 22 und noch bevorzugter 1 bis 12 Kohlenstoffatome aufweist;
- die Gruppe R₁₃ ist eine geradkettige oder verzweigte Alkylgruppe, die unter den für R₁₂ angegebenen Gruppen ausgewählt ist, oder bedeutet eine einwertige Gruppe mit Estergruppe, die der folgenden Formel (VII) entspricht: 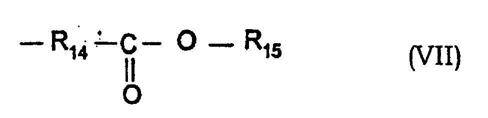 worin die Gruppen R₁₄ und R₁₅, die identisch oder voneinander verschieden sein können, Kohlenwasserstoffgruppen, vorzugsweise vom Alkyltyp, mit 1 bis 12 Kohlenstoffatomen und vorzugsweise 1 bis 8 Kohlenstoffatomen bedeuten.

14. Zusammensetzung nach einem der vorhergehenden Ansprüche, **dadurch gekennzeichnet, daß** die amidierte Verbindung das Ethyl-N-butyl-N-acetyl-aminopropionat ist.

15. Zusammensetzung nach einem der vorhergehenden Ansprüche, **dadurch gekennzeichnet, daß** die amidierte Verbindung in der Zusammensetzung in einem Mengenanteil von 0,01 bis 50 Gew.-%, bezogen auf das Gesamtgewicht der Zusammensetzung, und vorzugsweise 0,1 bis 30 Gew.-%, bezogen auf das Gesamtgewicht der Zusammensetzung, vorliegt.

16. Zusammensetzung nach einem der vorhergehenden Ansprüche, **dadurch gekennzeichnet, daß** sie in Form einer Öl-in-Wasser-Emulsion vorliegt.

17. Verwendung einer amidierten Verbindung nach einem der Ansprüche 1, 12, 13 oder 14 in kosmetischen und/oder dermatologischen Zusammensetzungen, die ein Dibenzoylmethanderivat nach einem der Ansprüche 1, 9 oder 10 in Kombination mit mindestens einem 1,3,5-Triazinderivat nach einem der Ansprüche 1 bis 7 enthalten, um in diesen Zusammensetzungen die Stabilität des 1,3,5-Triazinderivats gegenüber UV-Strahlung zu verbessern.

18. Verfahren zur Verbesserung der Stabilität von kosmetischen und/oder dermatologischen Zusammensetzungen gegenüber UV-Strahlung, die ein Dibenzoylmethanderivat nach einem der Ansprüche 1, 9 oder 10 und ein 1,3,5-Triazinderivat nach einem der Ansprüche 1 bis 7 enthalten, **dadurch gekennzeichnet, daß** es darin besteht, in die Zusammensetzungen eine amidierte Verbindung nach einem der Ansprüche 1, 12, 13 oder 14 in einer wirksamen Menge einzuarbeiten.

## Claims

1. Cosmetic and/or dermatological composition comprising, in a cosmetically and/or dermatologically acceptable support:
- i) at least one 1,3,5-triazine derivative corresponding to formula (I) below: 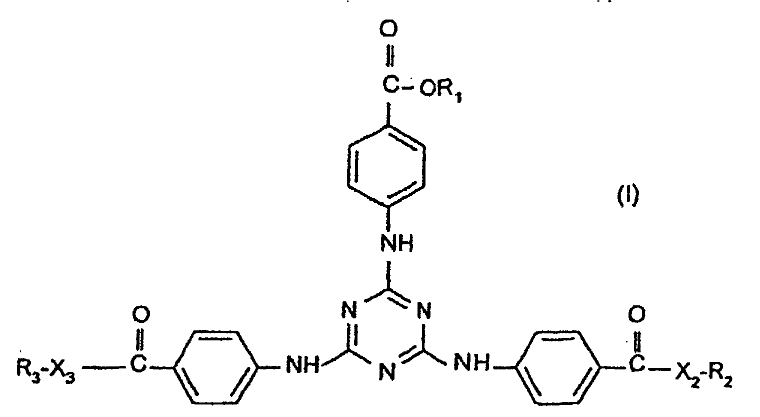 in which:
- X₂ and X₃, which may be identical or different, represent oxygen or an -NH- radical;
- R₁, R₂ and R₃, which may be identical or different, are chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a polyoxyethylenated radical comprising from 1 to 6 ethylene oxide units and whose terminal OH group is methylated; a radical of formula (II), (III) or (IV) below: 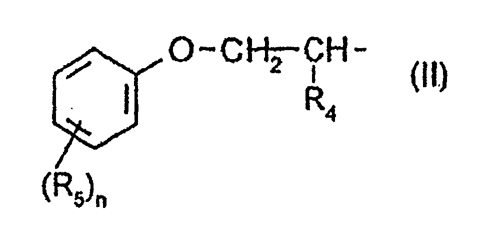 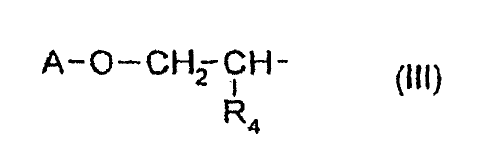 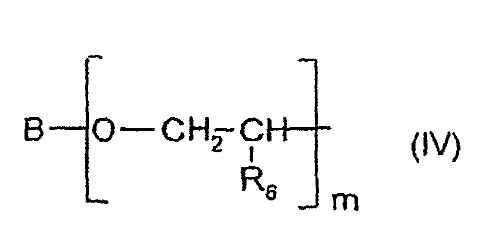
in which:
- R₄ is hydrogen or a methyl radical;
- R₅ is a C₁-C₉ alkyl radical;
- n is an integer ranging from 0 to 3;
- m is an integer ranging from 1 to 10;
- A is a C₄-C₈ alkyl radical or a C₅-C₈ cycloalkyl radical;
- B is chosen from: a linear or branched C₁-C₈ alkyl radical; a C₅-C₈ cycloalkyl radical; an aryl radical optionally substituted with one or more C₁-C₄ alkyl radicals;
- R₆ is hydrogen or a methyl radical,
- ii) at least one dibenzoylmethane derivative corresponding to formula (V) below: 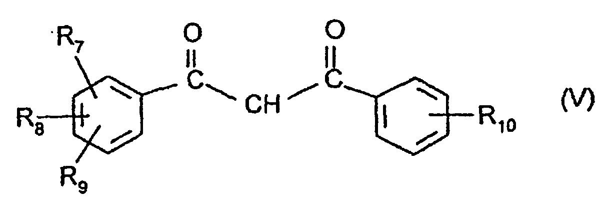 in which R₇, R₈, R₉ and R₁₀, which may be identical or different, independently represent hydrogen or a hydroxyl radical or a linear or branched C₁-C₈ alkyl radical or a linear or branched C₁-C₈ alkoxy radical,
- and iii) at least one amide compound.

2. Composition according to Claim 1, **characterized in that** the 1,3,5-triazine derivative is chosen from those of formula (I) having all of the following characteristics:
- X₂ and X₃ are identical and represent oxygen;
- R₁ is chosen from: a C₅-C₁₂ cycloalkyl radical optionally substituted with one or more C₁-C₄ alkyl radicals; a radical of formula (II), (III) or (IV) in which:
- B is a C₁-C₄ alkyl radical;
- R₆ is a methyl radical;
- R₂ and R₃, which may be identical or different, are chosen from: hydrogen; an alkali metal; an ammonium radical optionally substituted with one or more alkyl or hydroxyalkyl radicals; a linear or branched C₁-C₁₈ alkyl radical; a C₅-C₁₂ cycloalkyl radical optionally substituted with one or more C₁-C₄ alkyl radicals; a radical of formula (II), (III) or (IV) in which:
- B is a C₁-C₄ alkyl radical;
- R₆ is a methyl radical.

3. Composition according to Claim 1, **characterized in that** the 1,3,5-triazine derivative is chosen from those of formula (I) having all of the following characteristics:
- X₂ and X₃ are identical and represent the -NH-radical;
- R₃ is chosen from: a linear or branched C₁-C₁₈ alkyl radical; a C₅-C₁₂ cycloalkyl radical optionally substituted with one or more C₁-C₄ alkyl radicals;
- R₁ is chosen from: hydrogen; an alkali metal; an ammonium radical; a radical of formula (IV); a linear or branched C₁-C₁₈ alkyl radical; a C₅-C₁₂ cycloalkyl radical optionally substituted with one or more C₁-C₄ alkyl radicals;
- R₂ is chosen from: a linear or branched C₁-C₁₈ alkyl radical; a C₅-C₁₂ cycloalkyl radical optionally substituted with one or more C₁-C₄ alkyl radicals.

4. Composition according to Claim 1, **characterized in that** the 1,3,5-triazine derivative is chosen from those having all of the following characteristics:
- X₂ is oxygen;
- X₃ is the -NH- radical;
- R₃ is chosen from: a linear or branched C₁-C₁₈ alkyl radical; a C₅-C₁₂ cycloalkyl radical optionally substituted with one or more C₁-C₄ alkyl radicals;
- R₁ is chosen from: hydrogen; an alkali metal; an ammonium radical; a radical of formula (IV); a linear or branched C₁-C₁₈ alkyl radical; a C₅-C₁₂ cycloalkyl radical optionally substituted with one or more C₁-C₄ alkyl radicals;
- R₂ is chosen from: hydrogen; an alkali metal; an ammonium radical; a radical of formula (IV); a linear or branched C₁-C₁₈ alkyl radical; a C₅-C₁₂ cycloalkyl radical optionally substituted with one or more C₁-C₄ alkyl radicals.

5. Composition according to Claim 4, **characterized in that** the 1,3,5-triazine derivative is that corresponding to the following formula: 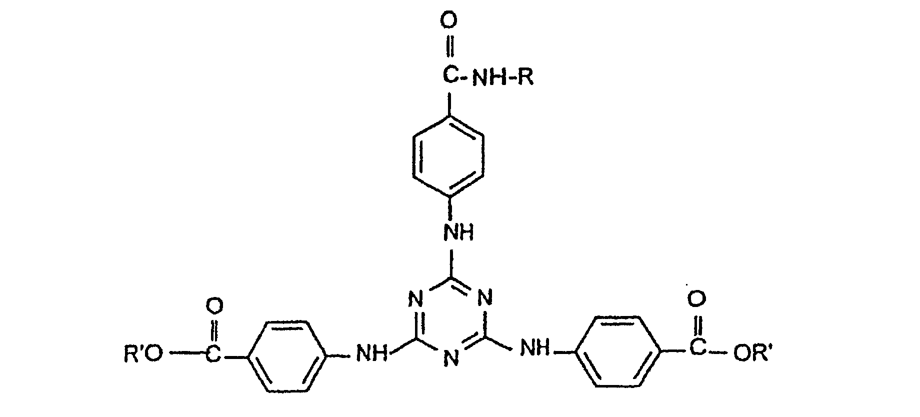 in which R' denotes a 2-ethylhexyl radical and R denotes a tert-butyl radical.

6. Composition according to Claim 1, **characterized in that** the 1,3,5-triazine derivative is chosen from those having all of the following characteristics:
- X₂ and X₃ are identical and represent oxygen;
- R₁, R₂ and R₃ are identical and represent a C₆-C₁₂ alkyl radical or a polyoxyethylenated radical comprising from 1 to 6 ethylene oxide units and in which the terminal OH group is methylated.

7. Composition according to Claim 6, **characterized in that** the 1,3,5-triazine derivative is that corresponding to the following formula: 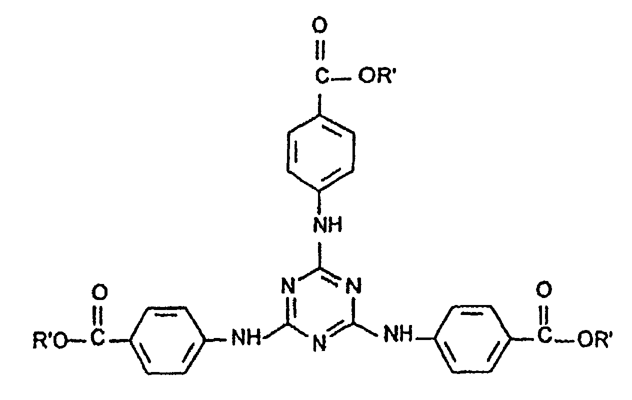 in which R' denotes a 2-ethylhexyl radical.

8. Composition according to any one of Claims 1 to 7, **characterized in that** the 1,3,5-triazine derivative is present in the composition in a content ranging from 0.5% to 20% by weight, relative to the total weight of the composition, preferably from 1% to 10% by weight, relative to the total weight of the composition.

9. Composition according to any of Claims 1 to 8, **characterized in that** the dibenzoylmethane derivative is chosen from:
- 2-methyldibenzoylmethane,
- 4-methyldibenzoylmethane,
- 4-isopropyldibenzoylmethane,
- 4-tert-butyldibenzoylmethane,
- 2,4-dimethyldibenzoylmethane,
- 2,5-dimethyldibenzoylmethane,
- 4,4'-diisopropyldibenzoylmethane,
- 4-tert-butyl-4'-methoxydibenzoylmethane,
- 2-methyl-5-isopropyl-4'-methoxydibenzoyl-methane,
- 2-methyl-5-tert-butyl-4'-methoxydibenzoyl-methane,
- 2,4-dimethyl-4'-methoxydibenzoylmethane,
- 2,6-dimethyl-4-tert-butyl-4'-methoxydibenzoylmethane,
- 4,4'-dimethoxydibenzoylmethane.

10. Composition according to any one of the preceding claims, **characterized in that** the dibenzoylmethane derivative is 4-tert-butyl-4'-methoxydibenzoylmethane.

11. Composition according to any one of the preceding claims, **characterized in that** the dibenzoylmethane derivative is present in the composition in a content ranging from 0.2% to 15% by weight, relative to the total weight of the composition, preferably from 0.2% to 10% by weight, relative to the total weight of the composition.

12. Composition according to any one of the preceding claims, **characterized in that** the amide compound is chosen from those corresponding to formula (VI) below: 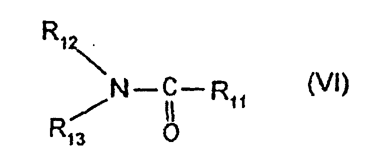 in which the radicals R₁₁, R₁₂ and R₁₃, which may be identical or different, independently represent hydrogen or monovalent, saturated or unsaturated, aliphatic, cycloaliphatic or cyclic, optionally functionalized hydrocarbon radicals containing from 1 to 30 carbon atoms, preferably from 1 to 22 carbon atoms, limits included, it being understood that, in this formula, the radical R₁₁ can form, with the radical R₁₂ or with the radical R₁₃, a ring containing from 5 to 18 carbon atoms inclusively, and that the radicals R₁₂ and R₁₃ can together form a ring containing from 5 to 18 carbon atoms, limits included.

13. Composition according to Claim 12, **characterized in that** the amide compound is chosen from those having at least one, and even more preferably all, of the following characteristics:
- the amide compound is an N-substituted amide, and even more preferably N,N-disubstituted,
- R₁₁ is a linear or branched, preferably C₁-C₂₂ and even more preferably C₁-C₁₂, alkyl radical, or alternatively a phenyl radical which is itself optionally substituted with one or more linear or branched C₁-C₁₂ alkyl radicals,
- R₁₂ is a linear or branched, preferably C₁-C₂₂ and even more preferably C₁-C₁₂, alkyl radical,
- R₁₃ is a linear or branched alkyl radical chosen from those defined for R₁₂, or alternatively represents a monovalent radical with an ester function corresponding to formula (VII) below: 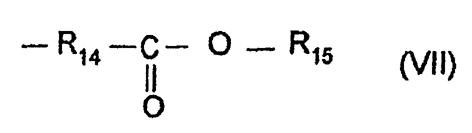 in which R₁₄ and R₁₅, which may be identical or different, represent two hydrocarbon radicals, preferably of alkyl type, containing from 1 to 12 carbon atoms, preferably from 1 to 8 carbon atoms.

14. Composition according to any one of the preceding claims, **characterized in that** the amide compound is ethyl N-butyl-N-acetylaminopropionate.

15. Composition according to any one of the preceding claims, **characterized in that** the amide compound is present in the composition in a content ranging from 0.01% to 50% by weight relative to the total weight of the composition, preferably from 0.1% to 30% by weight relative to the total weight of the composition.

16. Composition according to any one of the preceding claims, **characterized in that** it is in the form of an oil-in-water emulsion.

17. Use of an amide compound as defined in any one of Claims 1, 12, 13 or 14, in cosmetic and/or dermatological compositions containing a dibenzoylmethane derivative as defined in any one of Claims 1, 9 or 10, in combination with at least one 1,3,5-triazine derivative as defined in any one of Claims 1 to 7, in order to enhance the stability to UV radiation of the said 1,3,5-triazine derivative in the said compositions.

18. Process for enhancing the stability to UV radiation of the cosmetic and/or dermatological compositions comprising a dibenzoylmethane derivative as defined in any one of Claims 1, 9 or 10, and a 1,3,5-triazine derivative as defined in any one of Claims 1 to 7, **characterized in that** it consists in introducing into the said compositions an effective amount of an amide compound as defined in any one of Claims 1, 12, 13 or 14.
